# EUROPEAN PATENT SPECIFICATION

(11) **EP 1 595 552 B1**
(45) Date of publication and mention of the grant of the patent: **25.06.2008**
(21) Application number: 04076393.0
(22) Date of filing: 10.05.2004
(51) Int. Cl.: A61L 9/12

(54) **Air-freshener for a vehicle**
Lufterfrischungsgerät für Autos
Dispositif pour rafraîchir de l'air dans une voiture

(43) Date of publication of application: 16.11.2005
(73) Proprietor: Sara Lee/DE N.V., 3532 AA Utrecht (NL)
(72) Inventor: Bulsink, Dirk Jan, 2331 BZ Leiden (NL)
(74) Representative: Winckels, Johannes Hubertus F.

(56) References cited:
- EP-A- 1 031 446
- WO-A-90/04339
- WO-A-03/028775
- US-A- 4 621 768
- US-A- 5 437 410

## Description

The invention relates to an air-freshener for a vehicle, in particular a motorcar, provided with a housing into which a container for a volatile liquid or a liquid containing one or more volatile components can be inserted, in the opening of which container a wick extending into the housing is fitted, and with a movable covering cap which, when the container with wick has been inserted into the housing, can cover the wick entirely or partially, and with a control member for setting the position of the covering cap.

EP1031446 discloses an air-freshener showing the features of the preamble, wherein a cap is axially moved along a wick thereby uncovering the wick, In this way, the wick is exposed and the wick, soaked by a fragrance contained in a bottle, can release the fragrance for providing a pleasant ambient atmosphere to the car and/or to expel odours that are undesired Although the disclosed air-freshener is easily handed and small enough to attach in the car interior without problems, a desire exists to further minimize the disclosed apparatus.

Therefore, it is an object of the invention to provide a further minimized air-freshener, that provides a covering cap with adequate sealing of the wick in a covering position and that provides a proper and controllable distribution of fragrance in an uncovered position.

In an aspect, for attaining the above described objects, the invention provides an air freshener according to the features of claim 1.

By providing an air freshener, wherein the wick is covered by a sideways movable covering cap, dimensions of the air-freshener can be shortened considerably, since it is no longer necessary to provide room for elevating the covering member. By slight sideways movement, the wick is already uncovered and is able to distribute fragrance.

WO 03/028775 discloses a fragrance dispenser having a fan that passes air over a wicked single or double fragrance. In an embodiment, the size of duct openings is controlled by a respective shutter assembly, comprising a shutter connected to a peg which is slidably movable in a vertical slot in the front cover of the dispenser.

US 5,437,410 discloses a dispenser having a housing, wherein a second cylinder section slides within grooves of a first half cylinder to dispose a diffuser to ambient air currents or to seal the diffuser therefrom.

In a preferred embodiment, the covering cap comprises a first semi-open part that closes onto a second semi-open part. Further, preferably, a sliding mechanism is provided for slidingly moving the first part to and from said second part, wherein the control member comprises a knob for slidingly moving said first cap. In one embodiment, the second semi-open part may be fixed to the housing.

The housing may provided with ventilating openings via which, if a wick is arranged to extend in the housing, air can flow along the entirely or partially released wick. In an embodiment, wherein the first part is arranged to close the ventilation opening when covering the wick, vaporizing of the wick during covering is further minimized by providing a covering cap, wherein air circulation around the covering cap is minimized. Further ventilating openings may be provided in the wall of the housing.

In a further preferred embodiment, a fastening element may be present for securing the air-freshener on a strip-shaped element, in particular that of a guard of the fan in the dashboard of a motorcar. When the air-freshener is connected to the fan of the vehicle, the air flow through the air-freshener may be controllable both by setting the position of the covering cap, and by switching the fan of the vehicle faster or slower and/or by switching the temperature of the air flow in the vehicle higher or lower. In addition the housing may comprise a holding element for retaining an insertable and removable container for a volatile liquid or a liquid having one or more volatile components. Said holding element may be a flexible lip.

According to an embodiment, the container comprises a wick extending from an opening of said container and a detachable covering cap for covering the wick wherein said covering cap having a dimension smaller than the wick so as to compress the wick while being covered.

Further features and benefits related to the invention are illustrated in the drawings. Herein.
Figure 1 shows a front perspective schematic view of an embodiment of the invention;
Figure 2 shows a rear perspective schematic view of an embodiment of the invention;
Figure 3 shows a schematic sectional view along a line I-I in Figure 1 of the covering cap in closed position;
Figure 4 shows a schematic sectional view along a line I-I in Figure 1 of the covering cap in fully opened position; and
Figure 5 schematically shows the embodiment of Figure 1 in an exploded view.

In the figures, the same or corresponding items are referenced by the same reference numerals

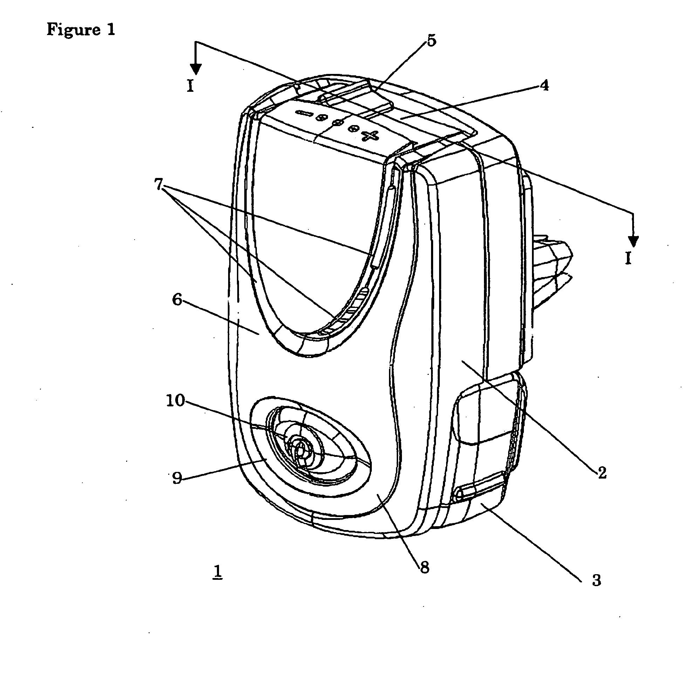

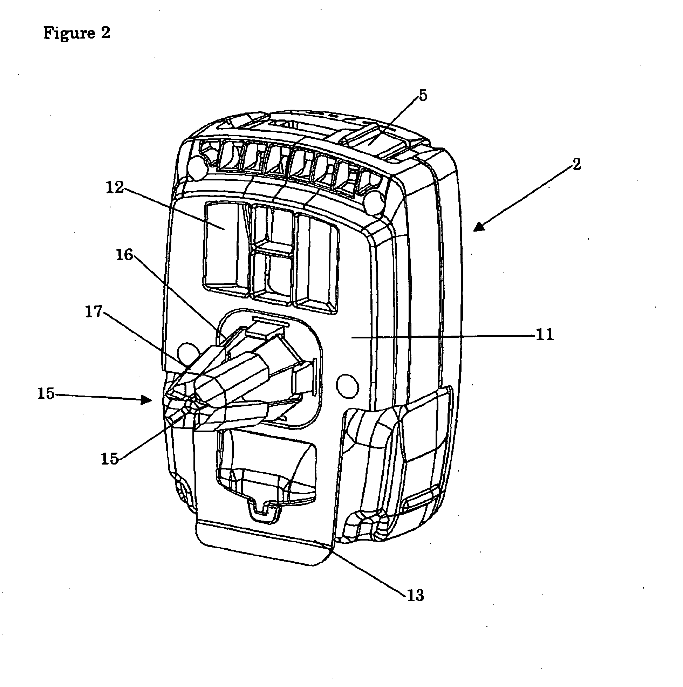

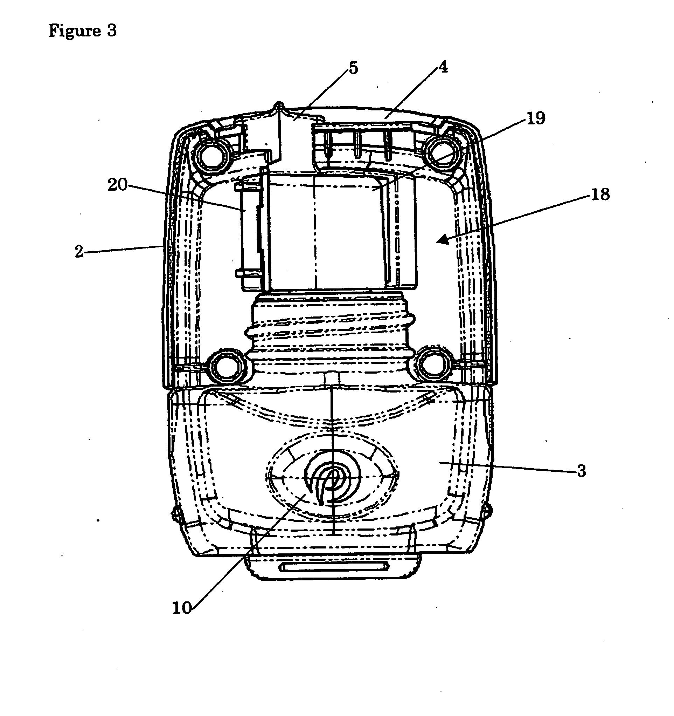

In Figure 1 a front perspective schematic view of an embodiment of the air freshener according to the invention is illustrated. The air-freshener 1 is attachable to a site in the interior of a vehicle, in particular a motorcar, in a way further described with reference to Figure 2. The air-freshener 1 is provided with a housing 2 into which a container 3 for a volatile liquid is inserted. A sliding mechanism 4 is present for slidingly moving a knob 5 that opens or closes a covering cap as will be further described with reference to Figure 3. The air-freshener comprises an interchangeable front plate 6 that can be adapted to a custumers favourite colour or appearance. The front plate 6 comprises ventilation outlets 7 via which, when a wick is arranged to extend in the housing 2, air can flow along the entirely or partially released wick.

The front plate further comprises an extending lid 8 comprising an opening 9 that matches to a protrusion 10 that is provided on the container 3. The air outlets 7 are oriented partially sideways, so that fragrance is ventilated into a broad range of forward directions.

Figure 2 shows a rear perspective schematic view of the air freshener illustrated in Figure 1. The rear face 11 comprises a ventilation opening section 12, that allows air to be transported through the housing 2, so that air can flow along the entirely or partially released wick (not shown). The rear face is further provided with a flexible fixation clip 13 that snaps onto a protrusion that is provided on the container 3 on the rear side. In this way, the container is partially introduced into the housing 2, until snap fit of the clip 13. Furthermore, a fastening element 14 is present for securing the air-freshener on a strip-shaped element, in particular that of a guard of the fan in the dashboard of a motorcar. The fastening element 14 comprises four clamping elements 15 projecting outwards from the housing 2, which clamping elements 15 are separated from each other according to two planes which are at least substantially perpendicular to each other, to enable securing the air-freshener 1 either on a vertical, or on a horizontal strip-shaped element. The clamping elements 15 are formed by metal strips 16 provided, at least at the ends thereof, with a plastic covering layer 17.

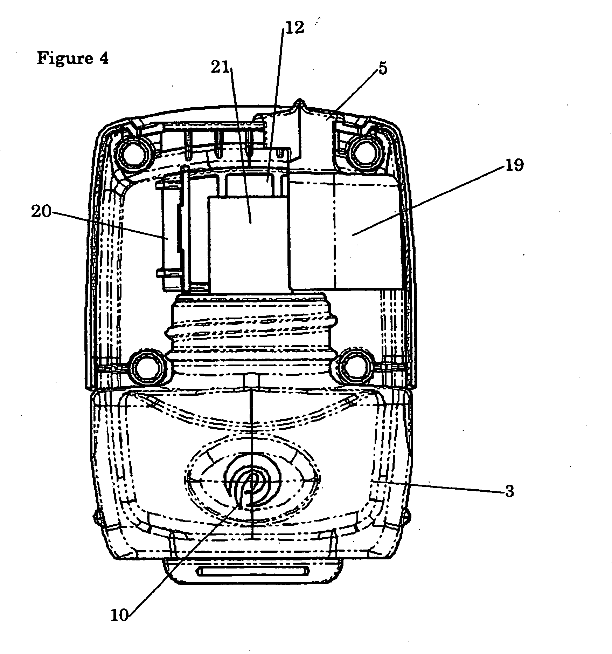

Figure 3 shows a schematic sectional view along a line I-I in Figure 1. In Figure 3, the covering cap 18 is illustrated in closed position. Here the wick (see Figure 4) is completely covered by the covering cap 18, so that the wick can not vaporize fragrances contained in the container 3. The covering cap 18 comprises a first semi-open part 19 that closes onto a second semi-open part 20. The second semi-open part 20 is fixed to the housing 2. First cover part 19 is connected to a knob 5 that can move along a sliding mechanism 4. Through this sliding movement the cover part 19 is sideways movable from the wick, so as to uncover said wick as is illustrated in Figure 4.

Figure 4 shows the embodiment of Figure 3 in fully opened position of the covering cap 18. The wick 21 is now uncovered and ventilation is provided via the ventilation openings 12 along the entirely or partially released wick 213. By arranging the covering cap 18 according to the invention, the apparatus can effectively be dimensioned according to the dimension of the container 3 comprising the wick 213, and no extra latitude is needed for providing axial movement to the cap 18.

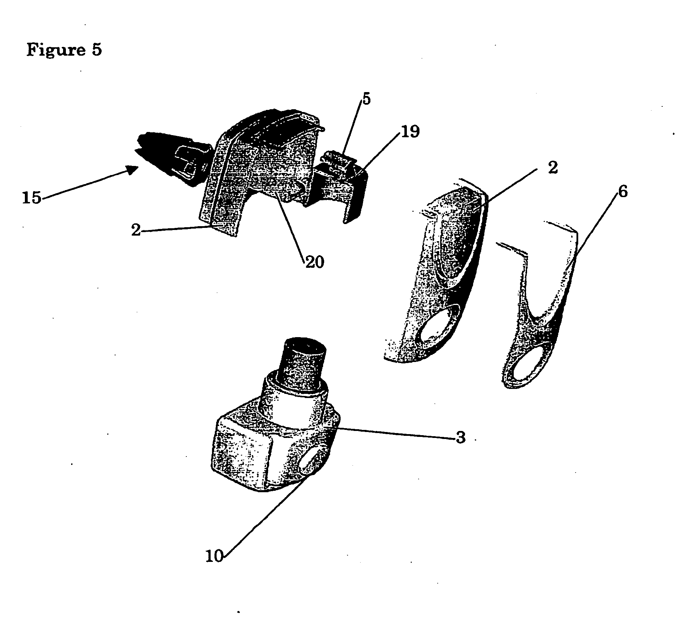

Figure 5 schematically shows the embodiment of Figure 1 in an exploded view. In use, the rear part 22 and front part 213 of the housing are connected. The front plate 6 however can be clipped interchangeably to the front part 25 so that the user can modify the appearance of the air-freshener to correspond to his particular preference. Furthermore, the container 3 comprising the wick 21 can be of a refill type or of a replacement type and is specifically adapted to match the clip element 13 described with reference to Figure 2. The front plate 6 comprises a see-through opening for identifying the fragrance level in the container.

Although in the drawings the covering cap comprises a fixed part and a slidable part, other embodiments may comprises a covering cap that slides or splits the covering cap. Such variations are deemed to fall within the scope of the attached claims.

## Claims

1. An air-freshener for a vehicle, in particular a motorcar, provided with a housing (2) into which a container (3) for a volatile liquid or a liquid containing one or more volatile components can be inserted in use, in the opening of which container (3) a wick (21) extending into the housing (2) is fitted, and with a movable covering cap (18) which, when the container (3) with wick has been inserted into the housing (2), can cover the wick entirely or partially, and with a control member for setting the position of the covering cap (18) **characterized in that** the wick (21) is covered by a sideways movable covering cap (18).

2. An air-freshener according to claim 1, **characterized in that** the covering cap (18) comprises a first semi-open part (19) that closes onto a second semi-open part (20).

3. An air-freshener according to claim 2, further comprising a sliding mechanism (4) for slidingly moving the first part (19) to and from said second part (20), the control member comprising a knob (5) for slidingly moving said first cap.

4. An air-freshener according to claim 2 or 3, **characterized in that** the second semi-open (20) part is fixed to the housing (2).

5. An air-freshener according to any of the preceding claims, **characterized in that** the housing (2) is provided with ventilating openings (12) via which, if a wick (21) is arranged to extend in the housing (2), air can flow along the entirely or partially released wick.

6. An air-freshener according to any of claims 2-4 in combination with claim 5, wherein the first part (19) is arranged to close the ventilation opening (12) when covering the wick (21).

7. An air-freshener according to claim 5 or 6, **characterized in that** further ventilating opening (7) are provided in the wall of the housing (2).

8. An air-freshener according to any one of the preceding claims, **characterized in that** a fastening element (14) is present for securing the air-freshener on a strip-shaped element, in particular that of a guard of the fan in the dashboard of a motorcar.

9. An air-freshener according to claim 8, **characterized in that** when the air-freshener is connected to the fan of the vehicle, the air flow through the air-freshener is controllable both by setting the position of the covering cap (18), and by switching the fan of the vehicle faster or slower and/or by switching the temperature of the air flow in the vehicle higher or lower.

10. An air-freshener according to any one of the preceding claims, **characterized in that** the housing (2) comprises a holding element for retaining an insertable and removable container (3) for a volatile liquid or a liquid having one or more volatile components.

11. An air-freshener according to any of the preceding claims, wherein the housing (2) further is provided with an clippable face plate provided with a see-through opening for identifying the liquid level in the container (3).

## Patentansprüche

1. Lufterfrischer für ein Fahrzeug, insbesondere einen Kraftwagen, versehen mit einem Gehäuse (2), in das bei Gebrauch ein Behälter (3) für eine flüchtige Flüssigkeit oder eine Flüssigkeit, die einen oder mehr flüchtige Bestandteile enthält, eingesetzt werden kann, wobei in die Öffnung des Behälters (3) ein Docht (21), der sich in das Gehäuse (2) erstreckt, eingepasst wird, und mit einer bewegbaren Abdeckkappe (18), die, wenn der Behälter (3) mit Docht in das Gehäuse (2) eingesetzt worden ist, den Docht ganz oder teilweise abdecken kann, und mit einem Steuerelement zum Einstellen der Position der Abdeckkappe (18) **dadurch gekennzeichnet, dass** der Docht (21) durch eine seitwärts bewegbare Abdeckkappe (1 8) abgedeckt wird.

2. Lufterfrischer nach Anspruch 1 , **dadurch gekennzeichnet, dass** die Abdeckkappe (18) einen ersten halboffenen Teil (19) umfasst, der sich auf ein zweites halboffenes Teil (20) schließt.

3. Lufterfrischer nach Anspruch 2, ferner umfassend einen Schiebemechanismus (4), um den ersten Teil (19) schiebend auf den zweiten Teil (20) zu und davon weg zu bewegen, wobei das Steuerelement einen Knauf (5) zum schiebenden Bewegen der ersten Kappe aufweist.

4. Lufterfrischer nach Anspruch 2 oder 3, **dadurch gekennzeichnet, dass** der zweite halboffene (20) Teil an dem Gehäuse (2) befestigt ist.

5. Lufterfrischer nach irgendeinem der vorangehenden Ansprüche, **dadurch gekennzeichnet, dass** das Gehäuse (2) mit Ventilationsöffnungen (12) versehen ist, durch die, falls ein Docht (21) angeordnet ist, sich in das Gehäuse (2) zu erstrecken, Luft entlang des ganz oder teilweise gelösten Dochtes strömen kann.

6. Lufterfrischer nach irgendeinem der Ansprüche 2 bis 4 in Kombination mit Anspruch 5, wobei der erste Teil (19) angeordnet ist, die Ventilationsöffnung (12) zu verschießen, wenn er den Docht (21) abdeckt.

7. Lufterfrischer nach Anspruch 5 oder 6, **dadurch gekennzeichnet, dass** das weitere Ventilationsöffnungen (7) in der Wand des Gehäuses (2) vorgesehen sind.

8. Lufterfrischer nach irgendeinem der vorhergehenden Ansprüche, **dadurch gekennzeichnet, dass** ein Befestigungselement (14) vorhanden ist, um den Lufterfrischer auf einem leistenförmigen Element sicher zu befestigen, insbesondere dem einer Schutzvorrichtung des Gebläses in dem Armaturenbrett eines Kraftwagens.

9. Lufterfrischer nach Anspruch 8, **dadurch gekennzeichnet, dass**, wenn der Lufterfrischer mit dem Gebläse des Fahrzeugs verbunden ist, der Luftstrom durch den Lufterfrischer sowohl durch Einstellen der Position der Abdeckungskappe (18), als auch durch Schneller- oder Langsamerschalten des Gebläses des Fahrzeugs und/oder durch Höher- oder Tieferschalten der Temperatur des Luftstroms in dem Fahrzeug steuerbar ist.

10. Lufterfrischer nach irgendeinem der vorhergehenden Ansprüche, **dadurch gekennzeichnet, dass** das Gehäuse (2) ein Halteelement zum Halten eines einsetzbaren und entfernbaren Behälters (3) für eine flüchtige Flüssigkeit oder eine Flüssigkeit, die einen oder mehr flüchtige Bestandteile enthält, umfasst.

11. Lufterfrischer nach irgendeinem der vorangehenden Ansprüche, wobei das Gehäuse (2) ferner mit einer ansteckbaren Frontplatte, versehen ist, die mit einer durchsichtigen Öffnung zur Bestimmung des Flüssigkeitsniveaus in dem Behälter (3) versehen ist.

## Revendications

1. Rafraîchisseur d'air pour véhicule, en particulier une automobile, comportant un boîtier (2) dans lequel peut être inséré en fonctionnement un récipient (3) destiné à un liquide volatil ou à un liquide contenant un ou plusieurs composants volatils, dans l'ouverture du dit récipient (3) étant insérée une mèche (21) s'étendant dans le boîtier (2), et un capot de couverture mobile (18) qui, lorsque le récipient (3) contenant la mèche a été inséré dans le boîtier (2), peut couvrir la mèche entièrement ou partiellement, et un élément de commande destiné à régler la position du capot de couverture (18), **caractérisé en ce que** la mèche (21) est couverte par un capot de couverture mobile latéralement (18).

2. Rafraîchisseur d'air selon la revendication 1, **caractérisé en ce que** le capot de couverture (18) comprend une première partie mi-ouverte (19) qui se ferme sur une deuxième partie mi-ouverte (20).

3. Rafraîchisseur d'air selon la revendication 2, comprenant en outre un mécanisme de coulissement (4) destiné à déplacer par coulissement la première partie (19) vers et depuis la deuxième partie (20), l'élément de commande comprenant un bouton (5) pour déplacer par coulissement ledit premier capot.

4. Rafraîchisseur d'air selon la revendication 2 ou 3, **caractérisé en ce que** la deuxième partie mi-ouverte (20) est fixée au boîtier (2).

5. Rafraîchisseur d'air selon l'une quelconque des revendications précédentes, **caractérisé en ce que** le boîtier (2) est doté d'ouvertures de ventilation (12) par lesquelles, si une mèche (21) est disposée de façon à s'étendre dans le boîtier (2), de l'air peut circuler le long de la mèche entièrement ou partiellement libérée.

6. Rafraîchisseur d'air selon l'une quelconque des revendications 2 à 4 en combinaison avec la revendication 5, dans lequel la première partie (19) est agencée pour fermer l'ouverture de ventilation (12) lorsqu'elle recouvre la mèche (21).

7. Rafraîchisseur d'air selon la revendication 5 ou 6, **caractérisé en ce que** d'autres ouvertures de ventilation (7) sont ménagées dans la paroi du boîtier (2).

8. Rafraîchisseur d'air selon l'une quelconque des revendications précédentes, **caractérisé en ce qu'**un élément de fixation (14) est présent pour fixer le rafraîchisseur d'air sur un élément en forme de bande, en particulier celui d'un élément de garde de la soufflante dans le tableau de bord d' une automobile.

9. Rafraîchisseur d'air selon la revendication 8, **caractérisé en ce que**, lorsque le rafraîchisseur d' air est relié à la soufflante du véhicule, l' écoulement d' air à travers le rafraîchisseur d' air peut être commandé à la fois par le réglage de la position du capot de couverture (18) et en faisant passer la soufflante du véhicule à une vitesse plus rapide ou une vitesse plus lente et/ou en faisant passer la température de l'écoulement d'air dans le véhicule une valeur supérieure ou inférieure.

10. Rafraîchisseur d'air selon l'une quelconque des revendications précédentes, **caractérisé en ce que** le boîtier (2) comprend un élément de maintien destiné à retenir un récipient pouvant être inséré et enlevé (3) destiné à un liquide volatil ou à un liquide comportant un ou plusieurs composants volatils,

11. Rafraîchisseur d'air selon l'une quelconque des revendications précédentes, dans lequel le boîtier (2) comporte en outre une plaque frontale pouvant être encliquetée, comportant une ouverture d'inspection permettant de déterminer le niveau de liquide dans le récipient (3).
